# EUROPEAN PATENT SPECIFICATION

(11) **EP 0 291 519 B1**
(45) Date of publication and mention of the grant of the patent: **02.08.1995**
(21) Application number: 87907371.6
(22) Date of filing: 13.10.1987
(51) Int. Cl.: A61M 1/00

(54) **BLOOD COLLECTION SYSTEM AND METHOD**
VORRICHTUNG ZUM SAMMELN VON BLUT SOWIE ENTSPRECHENDES VERFAHREN
PROCEDE ET SYSTEME DE RECUPERATION DU SANG

(30) Priority: 15.10.1986 US 919346
(43) Date of publication of application: 23.11.1988
(60) Divisional application: 91200383.7
(73) Proprietor: BAXTER INTERNATIONAL INC. (a Delaware corporation), Deerfield Illinois 60015 (US); THE AMERICAN NATIONAL RED CROSS, Washington, DC 20006 (US)
(72) Inventor: LYSAGHT, Michael, J., Tower Lakes, Barrington, IL 60010 (US); BOGGS, Daniel, R., Vernon Hills, IL 60061 (US); RITGER, Philip, L., El Toro, CA 92630 (US); STROMBERG, Robert, R., Silver Spring, MD 20906 (US); FRIEDMAN, Leonard, I, Silver Spring, MD 20904 (US)
(74) Representative: MacGregor, Gordon
(86) International application number: US8702604
(87) International publication number: WO8802641

(56) References cited:
- EP-A- 0 115 835
- EP-A- 0 132 210
- DE-A- 1 961 761
- US-A- 2 502 081
- US-A- 3 647 117
- US-A- 3 655 123
- US-A- 3 895 741
- US-A- 3 902 490
- US-A- 4 191 182
- US-A- 4 381 775
- US-A- 4 479 762
- US-A- 4 526 515
- US-A- 4 551 136
- US-A- 4 551 139

## Description

### Technical Field

The invention pertains to the field of blood component collection. More particularly, the invention pertains to the collection of blood components, especially plasma, from donors using lighweight equipment capable of easy transport.

### Background of the Invention

The development of single needle, disposable blood collection systems has provided a safe, relatively inexpensive and accepted modality for collecting whole blood from donors. The whole blood (which is collected in units of 450 ml) is usually centrifugally separated into various therapeutic components, such as red blood cells, platelets, and plasma, for direct transfusion or further processing into other therapeutic products. Such systems have made possible large-scale collection of whole blood from volunteer donors at sites such as church halls, schools or offices remote from medical facilities. The availability whole blood collection systems suitable for volunteer donors is important, because it provides access to a relatively large pool of healthy individuals from which to draw needed supplies of whole blood components for life-saving or therapeutic purposes.

The conventional whole blood collection systems, familiar to and accepted by volunteer donors, can be used to collect plasma, as just described. However, such systems, by design, yield only approximately 250 to 300 ml of plasma per donor. Furthermore, such systems, by design, also take red blood cells from the donor. The donor must internally replace red blood cells before he or she can donate again. Current governmental regulations in the United States prescribe a waiting period of 56 days, during which time the red blood cell donor cannot give blood. Thus, such conventional whole blood collection systems are limited for the collection of relatively large pools of plasma from which the various therapeutic plasma proteins, such as albumin and AHF (anti-hemophilic factor), are obtained by a process called fractionation.

The collection of only plasma from volunteer donors, as opposed to the collection of whole blood, is not widespread. As a result, much of the plasma now collected for fractionation purposes comes from paid donors, not volunteer donors. It would be desirable to make the collection of plasma a volunteer-based activity to a much greater extent than it is currently.

Various methods are known for the collection of only plasma from a donor (also called plasmapheresis). For example, using a modification of the above-described whole blood collection system, a unit of whole blood is collected and separated by centrifugation into red blood cells and plasma. The plasma is retained, while the red blood cells are immediately returned to the donor. The process is then repeated, collecting additional plasma and returning the red blood cells. The result is the collection of 500 to 600 ml of plasma for fractionation purposes. Because red blood cells are returned to the donor, this process allows more frequent donation, often as frequently as twice per week. However, this process is time-consuming and, in part for this reason, does not appeal to volunteer donors. Furthermore, during the process, while the whole blood is being separated into red blood cells and plasma, the blood collection system (typically a series of integrally attached bags) is physically separated from the donor. Such physical separation requires procedures to minimize the risk of error when several donors are being processed simultaneously that one donor's red blood cells are not inadvertently returned to another donor. In addition, physical separation of the blood from the donor could potentially raise concerns in the collection staff of exposure to infectious agents in the collected blood if fluid drips or leaks occur.

On-line extracorporeal separation systems, in which the blood collection system is not physically separated from the donor during the collection procedure, are also known. These can be either batch or continuous systems. Such systems employ either centrifugal separators or membrane filters.

A centrifuge-based system is disclosed in US-A-3,655,123 and has two needles, an outflow needle and an inflow needle. Whole blood is drawn from a donor via the outflow needle. The whole blood fills a buffer bag. Blood from the buffer bag drains, under the force of gravity into a centrifuge. The system of the Judson et al. patent uses the centrifuge to separate blood components. The plasma can be collected in a container. The red blood cells can be returned to the donor via the inflow needle.

A membrane-based system is disclosed in US-A-4,191,182.

The systems of US-A-3,655,123 and US-A-4,191,182 require an external source of electrical energy. Further, the systems rely upon a variety of heavy and somewhat delicate electro-mechanical components, including pumps and the centrifuge that prevent it from being readily portable. Such systems that include pumps are often constant volume systems, in which a relatively constant volume of fluid is pumped through the system per unit of time. Constant volume systems suffer from the disadvantage that undesirable, over pressure conditions can occur if one of the fluid flow lines is crimped or partly closed. The systems also require that the donor be subjected either to two needle punctures (one in each arm) or a large bore double lumen needle, neither of which are favored by volunteer donors. Finally, since these relatively complex systems are intended to be coupled to one donor at a time, multiple simultaneous donations would be prohibitively expensive.

The known extracorporeal separation systems are therefore expensive, complex, not "donor friendly", and generally unsuited for portable operation.

One system of membrane collection suitable for portable operation has been described in EP-A-0114698 and, in this system, a unit of blood is withdrawn from a donor into a set containing a membrane filter, tubing and a sterile blood container (such as a conventional blood bag). The whole blood is first passed through the filter. The plasma flows through the membrane filter and is collected in a separate plasma container. The remainder of the blood unit, which had passed from the inlet to the outlet of the filter, is accumulated in the sterile container. It can then be immediately returned to the donor.

In this approach, the pressure available for driving the filtration process and for propelling the blood from the inlet to the outlet of the filter is relatively small. This pressure includes the donor's venous pressure (which, with an inflated pressure cuff on the donor's arm, is on the order of 40mm Hg) and available hydrostatic head (approximately 50mm Hg) for a total pressure on the order of 90mm Hg. These pressures may vary significantly from donor to donor. This can result in a relatively slow and variable plasma collection time. It also requires relatively large filters to function at the available low driving pressures. It can also be difficult to achieve precise anticoagulant flow proportional to blood flow with inexpensive and simple-to-use hardware.

Another membrane-based system is disclosed in a group of three United States Patents: US-A-4,479,760,

US-A-4,479,761 and US-A-4,479,762, all issued to Bilstad et al. The system of the Bilstad et al. patents utilizes a disposable module containing a hollow membrane filter, a plasma container and other elements. A fixture is provided to receive the module during the donation cycle. Constant volume pumps in the fixture are provided to draw whole blood from the donor into the inlet side of the filter and to return the concentrated red cells to the donor from the outlet side of the filter. A single needle is used from both drawing the whole blood from the donor and returning concentrated red blood cells to the donor.

The system of the Bilstad et al. patents requires an exterior source of electrical energy. In addition, the fixtures can be relatively expensive and complex.

EP-A-0132210 discloses a method of blood collection in which a pump is issued to draw blood from a donor through a separator. A blood component is collected in a variable volume container, which is pressurised to return the component through the separator to the donor.

DE-A-1961761 discloses a portable device for intravenous infusion.

EP-A-0115835 discloses a method of blood component collection and apparatus therefor, in which whole blood from a donor is drawn into a variable-volume pump chamber under negative pump pressure, and then forced through a separator by application of positive pressure to the chamber. Control of pressure through the separator is provided for by a throttle in an output from the separator. This arrangement, therefore, suffers the disadvantage previously referred to in relation to US-A-4191182 and US-A-3655123. The precharacterising parts of Claims 1 and 8 are based on EP-A-0115835.

The features distinguishing the invention from EP-A-0115835 are set out in the characterising parts of Claims 1 and 8.

The preferred apparatus of the invention is self-contained and does not require an external electrical connection or an external source of energy.

Other features and advantages of the present invention will become readily apparent from the following detailed description, the accompanying drawings, and the appended claims.

### Brief Description of the Drawings

FIGURE 1 is a perspective view of a plasmapheresis system, configured for use, in accordance with the present invention;
FIGURE 2 is a perspective view, partly broken away of a fixture, configured for transportation, in accordance with the present invention;
FIGURE 3 is a pictorial view illustrating portability of the fixture in Figure 2;
FIGURE 4 is a planar view of a blood contacting set in accordance with the present invention;
FIGURE 5 is a schematic diagram of an apparatus and method for plasmapheresis in accordance with the present invention;
FIGURE 6 is a flow diagram illustrating a method for plasmapheresis in accordance with the present invention;
FIGURE 7A is a side, schematic view of an apparatus for forcing whole blood out of the collection container;
FIGURE 7B is a front, schematic view of the apparatus of Figure 7A;
FIGURE 7C is a side, schematic view of a preferred apparatus for forcing whole blood out of the collection container; and
FIGURE 8 is a fragmentary side view, partly in section, of a filter usable with the system of the present invention.

### Detailed Description of the Preferred Embodiment

The present invention has many diverse embodiments. Several alternate embodiments are shown and described. However, it should be understood that the present disclosure is to be considered as an exemplification of the invention, and is not intended to limit the invention to the specific embodiments illustrated.

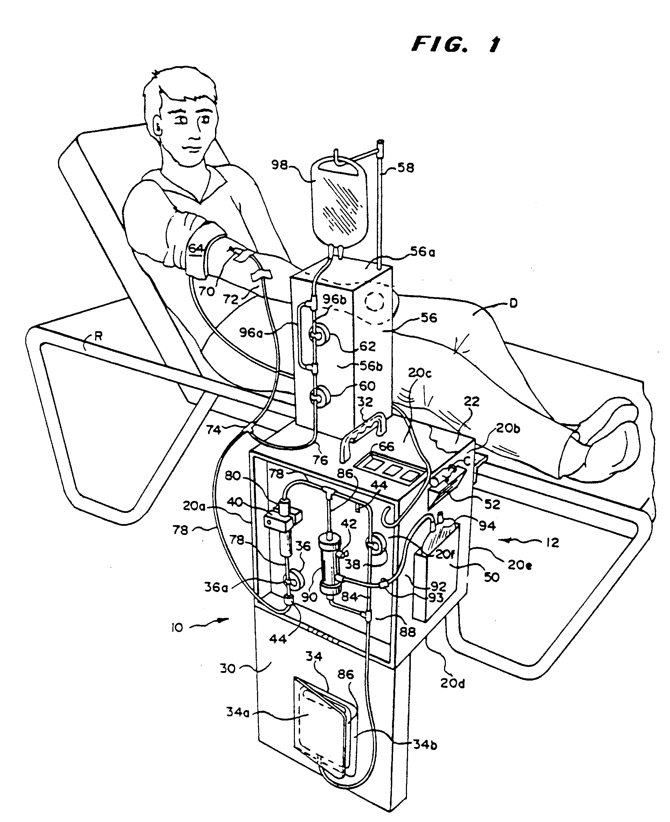

A self-contained, portable system 10 usable for the collection of blood components from donors is illustrated in Figures 1 to 3. In the particular embodiment, the system 10 is intended to collect plasma from volunteer donors. In this embodiment, the system 10 includes a portable, self-contained and reusable fixture 12. The system 10 also includes a single-use, disposable, integrally-formed tubing set 14. The set 14 is mounted in the fixture 12 during the collection process.

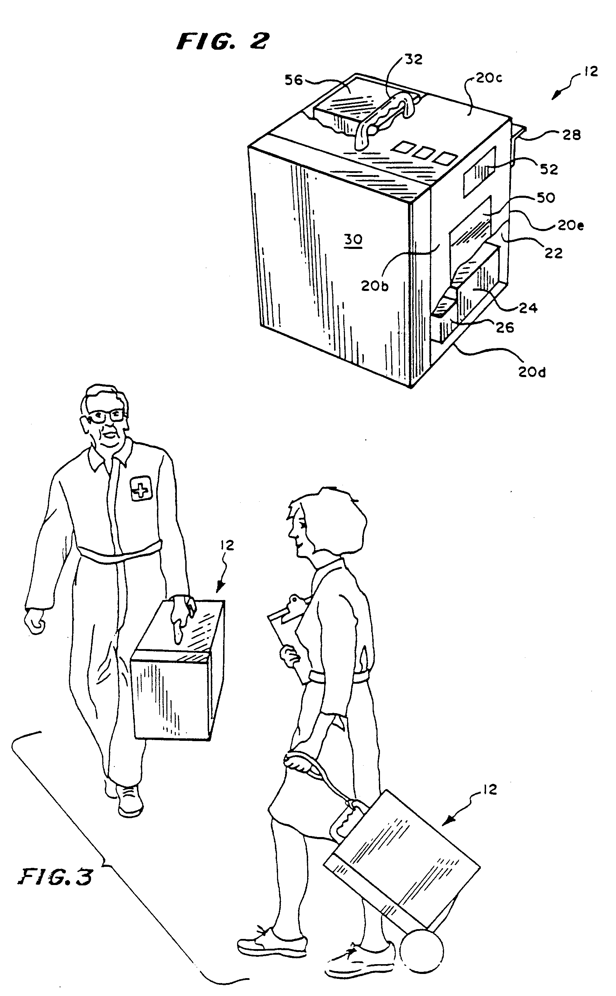

The fixture 12 includes a housing 20 which can be formed of a metal or plastic. The housing 20 has sides 20a, 20b; a top and bottom 20c, 20d and rear surface 20e. Housing 20 defines or forms a closed interior region 22 in which is located a control unit 24 (see Fig. 2).

Affixed to the rear surface 20e is a pair of clamps 28. The clamps 28 are intended to engage a rail R of a blood donation cot or bed, as shown in Fig. 1. Such cots are currently regularly used in connection with the collection of whole blood from volunteer donors.

The clamps 28 support the fixture 12 at an appropriate working height without any need for supporting legs or tables. The clamps 28 fold flat against the rear surface 20e for storage and transportation.

The housing 20 has a hinged front cover 30. During transportation, the cover 30 is closed and latched. The overall size of the fixture 12 when closed for the transportation and storage is on the order of 30 cm (12") wide by 30 cm (12") deep by 35 cm (14") high. A handle 32 is attached to the top surface 20c for use during transportation.

The hinged cover 30 holds, below the donor D, a whole blood collection container support or receptacle 34 with a hinged cover 34a. Located in the blood container support 34 is a force applying system 34b.

The fixture 12 also includes an energy source 51 for the force applying system 34b, as well as for the control system 24. The source 51 is self-contained in the housing 20, so that operation of the fixture 12 is independent of any external source of energy.

A recessed front panel 20f on the housing 20 supports clamps 36 and 38, a bubble sensor 40, a plasma separator support clamp 42 and tubing supports 44. The clamps 36, 38 are of a type conventionally used to close off flexible tubing members, and can take the form of pneumatically operated clamps. In this arrangement, in an unenergized condition, a spring biased clamping bar, such as the bar 36a, pinches the tubing in the clamp closed. When energized, by fluidic pressure, the clamping bar moves away from the tubing member permitting fluid to flow. The bubble sensor 40 is an ultrasonic sensor of a type conventionally used with blood donation and return systems to sense a gas-liquid interface. The sensor 40 is powered by a battery 26 housed within the interior region 22 (see Fig. 2).

Support 42 could be a spring clamp capable of removably supporting a cylindrical plasma separator such as a filter. Tubing supports 44 can correspond to small, L-shaped hangers of a type used to temporarily support flexible tubing.

Slidably affixed to the side 20b is a plasma container support 50. The support 50 can be a three-sided housing with a bottom but no top. Part of the side 20b forms the fourth side of the support 50. When the fixture 12 is being transported, the support 50 is pushed flat against the side 20b, as shown in Fig. 2.

While the energy source 51 can be variously constructed, in the embodiment shown in Figs. 1 to 5, the source 51 includes a hinged cover and receiver 52. The receiver 52 can be opened to receive a module containing a releasable charge of energy. In the illustrated embodiment, the module takes the form of a CO₂ cartridge C inserted in the fixture 12 to provide a modular, self-contained fluidic, charge of energy to actuate the fluidic control system 24, the force applying system 34b, as well as the clamps 36, 38, 60 and 62. Alternately, the module can take the form of a battery, either single use or rechargeable.

A pop-up column 56 extends from the top 20c. The column 56 supports an L-shaped, tubular, anticoagulant support member 58 at a top surface 56a. The L-shaped support member 58 provides a hanger for a container of anticoagulant solution. The pop-up column 56, on a front surface 56b supports two additional tubing clamps 60 and 62. The clamps 60, 62, as is discussed subsequently, are used to regulate the flow of anticoagulant when the system 10 is in use. For storage or transportation, the tubular support member 58 is retractable into the column 56. The column 56 is in turn pushed downward into the region 22. The top surface 56a then is positioned adjacent the top 20c of the fixture 12.

An inflatable cuff 64 is provided, coupled to the control unit and timer 24. A control panel 66 with a plurality of push buttons is positioned on the surface 20c.

Figure 2 illustrates the fixture 12 with the cover 30 closed and the column 56 retracted for storage and transportation. The fluidic control unit 24 and the battery 26 are also illustrated in Figure 2 positioned in the interior region 22. The hangers 28 can be closed flat against the surface 20e during transportation.

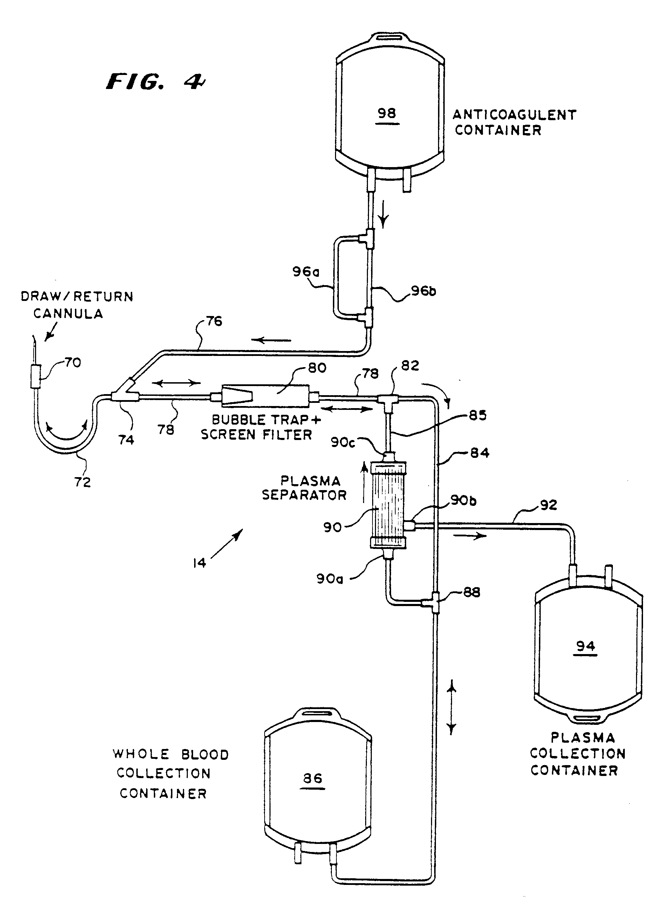

Figure 3 illustrates the portability of the fixture 12 when it is being taken to donation sites. The fixture 12 can be easily carried or pulled on a small cart of the type used to transport luggage. When the fixture 12 arrives at the donation site, it is mounted on the side of an available donor bed, as illustrated in Figure 1 and opened. The tubing set 14 can be mounted in the fixture 12. The modular CO₂ cartridge C can be inserted in the receiver 52, and used to energize the fixture 12. No additional exterior source of energy is needed to actuate the fluidic control system 24, the force applying system 34b, and the clamps 36, 38, 60 and 62 of the fixture 12 to carry out the donation process. Provision can be made in the fixture 12 for a storage region in which additional cartridges can be kept prior to use.

As an alternate to the column 56, the housing 20 can be elongated and the clamps 60, 62 can be mounted on the recessed surface 20f, above the tubing member 78. In this embodiment, the clamps 60, 62 would be spaced apart horizontally from one another.

As an alternate to the clamps 28, the fixture 12 can be fitted with a pair of foldable or telescoping rear legs. In this embodiment, the foldable front cover 30, when opened, can function as a front support. The fixture 12 in this embodiment will be self-supporting and will stand on the floor beside the donor bed.

Figure 4 illustrates details of the set 14. The set 14 includes a single lumen draw/return cannula or phlebotomy needle 70. The cannula 70 has pointed end that can be inserted into a vein of a donor D, to provide access to the donor's whole blood.

The cannula 70 is coupled to a flexible fluid flow conduit or tubular member 72. The member 72 is coupled to a Y-shaped junction 74. The junction 74 is in turn coupled to an anticoagulant delivering tubular member 76 and a tubular member 78. The member 78 alternately receives whole blood from the cannula 70 and returns concentrated red blood cells to the donor D.

A combined bubble trap and screen filter unit 80 is located in the line 78. The unit 80 is conventional device manufactured by Travenol Laboratories, Inc.

A T-shaped coupling member 82 couples the tubing member 78 to tubing members 84 and 85. Tubing member 84 is in fluid flow communication with a whole blood collection container 86. The container 86 is preferably a variable volume container, meaning that the interior volume of the container expands to accommodate the introduction of fluid and can be contracted to reduce the interior volume so as to expel or displace the fluid contents. The variable volume container 86 can correspond to a flexible, conventional, blood collection bag. It can also correspond to a rigid or semirigid container which includes a collapsible portion to reduce its interior volume.

A T-shaped coupling member 88 is located in the line 84. The member 88 also places the collection bag 86 into fluid flow communication with an inlet 90a of a blood component separator 90, which, in the illustrated embodiment, separates plasma from the other components of whole blood, notably red blood cells, white blood cells, and platelets. The plasma separator 90 can be implemented in a variety of ways. For example, and without limitation, the separator 90 could be implemented as a chromotography column, an electrophoretic apparatus, an immunoabsorbant column or a membrane filter. The filter could incorporate planar membrane sheets, or cylindrical membrane fibers, and can also include a means for rotating the filter to enhance its filtration efficiencies. In a preferred form of the invention, the plasma separator is implemented as an optimized, hollow fiber membrane filter.

Coupled to and in fluid flow communication with a plasma output port 90b, via a flexible tubular member 92 is a plasma collection container 94. The container 94 could be a flexible plastic container similar to the container 86. An outlet 90c of the separator 90 is coupled to the tubular member 85.

The anticoagulant delivery member 76 is coupled via tubular members 96a and 96b to a container 98 of anticoagulant solution. The member 96a has a smaller internal diameter than does the member 96b. By means of these two flow paths, the anticoagulant can be easily and cheaply metered into the blood being collected via the tubing member 78. The arrows on Figure 4 indicate directions of fluid flow when the set 14 is used with the fixture 12.

The tubing members of the set 14 can be formed of conventional, flexible plastic of a type suitable for contacting blood. The containers can be formed of conventional plastic now used in blood collection sets. Preferably, the set 14 comprises a sterile, integrally connected unit.

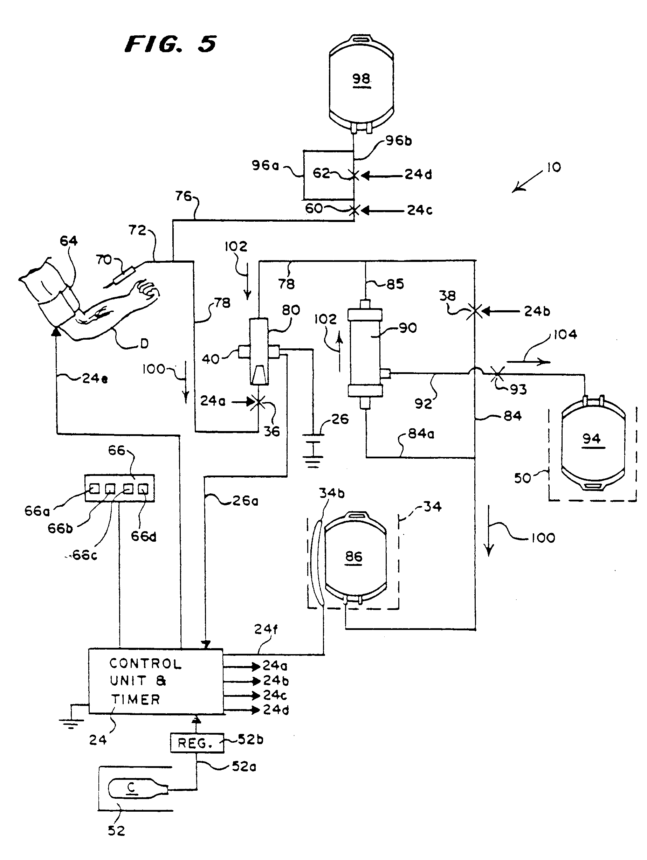

Figure 5 illustrates the system 10 schematically. The fluidic control unit and timer 24 are coupled to the fluidic source of energy C via a fluid flow input line 52a and a regulator 52b. The control unit and timer 24 are also coupled via a plurality of fluid flow lines 24a, 24b, 24c and 24d the fluid actuatable clamps 36, 38, 60 and 62, respectively. The unit 24 can selectively open each of the clamps 36, 38, 60 and 62 by providing fluidic energy on the respective line 24a, 24b, 24c and 24d. Fluidic line 24e couples the unit 24 to the inflatable cuff 64.

The bladder 34b can be inflated and deflated by the unit 24 via a fluidic control line 24f. The control unit and timer 24 receive electrical signals on the line 26a from the battery powered bubble detector 40. If the electrical signal on the line 26a indicates that a bubble has been detected in the line 78 during a return cycle, as discussed subsequently, the control unit 24 will permit clamp 36 to close thereby blocking any further flow in the line 78 to the donor D. An alarm condition can also be indicated on the panel 66.

The control unit and timer 24 can be implemented of standard fluidic logic components in accordance with the donor and return cycle described herein. The electrical signal on the line 26a can be coupled to a solenoid valve in the unit 24.

Figure 5 illustrates use of the mobile plasma collection system 10 in accordance with the present invention. The donor D is positioned adjacent to the sterile, sealed collection system 10. The system 10 includes the set 14 with the cannula 70, which could be a conventional, sterile single lumen phlebotomy needle of a type used in connection with blood collection. The needle 70 is coupled via flexible tubing 72, 78 and 84 of a conventional variety to the whole blood collection container or bag 86.

The collection bag 86 could be a flexible 500 ml plastic bag of a type now used for blood collection. The fluid operable clamp or valve 38 can be used to close off tubing member 84 under control of the unit 24. Closing the value 38 isolates the donor D from direct fluid flow communication from the container 86. Arrow 100 indicates the direction of flow of collected blood from the donor D into the collection bag 86. The whole blood drains from the donor D into the container or bag 86, as a result of the donor's internal blood pressure, which can be elevated in the region of the needle 70 by inflating the pressure cuff 64, as well as the force of gravity.

The container 86 is filled from the bottom as illustrated in Figures 1 and 5. Average fill time with a normal donor will be in a range of 4-7 minutes. The draw rate with an average donor will be in a range of 70-100 ml/minute.

Anticoagulant solution is metered from the container 98 through the two-part conduit 96a, 96b of known resistance. The anticoagulant solution is metered into the blood simultaneously with the whole blood being collected from the donor D.

The two tubes 96a and 96b each have a selected diameter and length. The tube 96a has a smaller diameter than does the tube 96b. By having both tubes 96a and 96b open simultaneously for a selected period of time and then closing one tube off while the other remains open, the rate of flow and quantity of anticoagulant mixed with the blood flowing through the member 72 can be regulated. Valve 62 can be used to close off the larger diameter conduit 96b under control of the unit 24. The dual tube system with members 96a, 96b makes it possible to keep the level of anticoagulant in the blood in the lumens of the tubing members, such as the member 78, and in the collection bag 86 between predetermined upper and lower limits even though donor blood rate is variable.

When the donor D has provided a unit of whole blood, the valves 60 and 38 are closed, and the inflatable cuff 64 is deflated. A force applying system 34b is then activated by the control unit 24. While the force applying unit can be variously constructed, in the illustrated embodiment, the force applying system takes the form of an inflatable bladder, is illustrated in Figure 5. The force applying system 34b can alternately be of a type that is mechanically or electrically activated, in which case the energy source 51 could take the form of a battery.

The generator 34b applies a force to the variable volume collection bag 86 to reduce its volume. The whole blood accumulated in the collection bag 86 is thus expressed or forced, through a conduit 84a into the plasma separator 90. The separator 90 will separate out 40-70% of the plasma in the whole blood passed through.

The whole blood passes through the separator 90 due to the force generator 34b in the direction 102. The plasma accumulates at the output port 90b and travels via the flexible tubing or conduit 92 to the plasma collection container 94. An arrow 104 indicates the direction of flow of the plasma.

The concentrated red blood cells, or residual blood component, exit from the separator 90 via the conduit 85, enter the conduit 78 and pass through the bubble trap and screen filter 80. The control unit 24 continuously monitors the electrical signal line 26a. In the event a bubble is detected in the trap 80, the clamp 36 is deenergized. Clamp 36, due to its internal spring biasing, immediately closes and blocks further fluid flow in the line 78 toward the donor D. An alarm condition can be indicated on the panel 66 and the operator can take corrective action.

If no bubbles are detected, the concentrated red blood cells will be returned to the donor D via the line 78 and the same single-lumen cannula 70 used for whole blood collection. During the collection phase and the separation/return phase, the donor D is continuously coupled to the system 10 by means of the single-lumen cannula 70 and the bi-directional fluid-flow conduit 72, 78. The collection of plasma occurs simultaneously with the return of the red blood cells.

Due to the features of the invention, the whole blood passes through the separator 90 for separation, and the red blood cells are returned to the donor solely in response to the force applied to the bag 86 by the generator 34b, and without the application of any additional external force.

When the whole blood collection bag 86 has been emptied, a four to seven minute process, the plasma has been collected in the bag 94, and the remaining concentrated red blood cells have been returned to the donor D. The valves 38, 60, 62 can then be opened and the process repeated. The return rate of concentrated red blood cells is in a range of 40-80 ml/minute. Because the bag 86 is filled and drained from the bottom, all whole blood is expressed from the container. Depending on the rate of collection of plasma in the bag 94 the process may be repeated two or three times.

The relatively low cost of the interconnected set 14 is an advantage of the system 10. The interconnected plastic members can be used in the collection of plasma from a single donor and then thrown away. In addition, since the bag 86 containing the collected blood remains continuously connected to the donor D, there is no chance that a donor D will accidentally receive the blood of another donor. Further, because the system 10 is continuously coupled to the donor D, the possibility of contamination is minimized.

The container 86 can be prefilled with sterile saline. The saline can be flushed from the container and the system 10 prior to the initiation of the initial blood collection cycle. Flushing or priming with saline insures a gas free system.

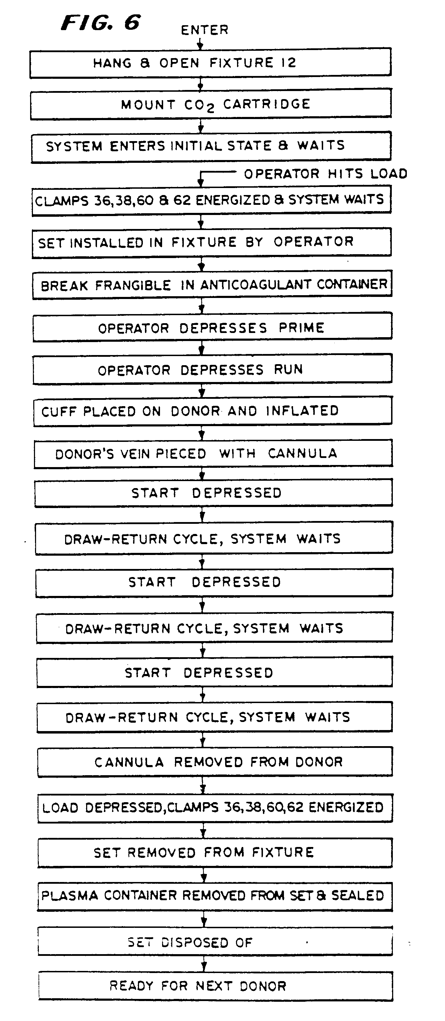

An overall operational sequence is illustrated in the flow diagram of Figure 6. The fixture 12 is hung on the donor bed the the cover 30 opened. A CO₂ cartridge C is removed from storage in the fixture 12 and inserted into the receptacle 52. This energizes the control unit and timer 24 which enters an initial state. When the operator is ready, the LOAD button 66a can be depressed. Upon sensing depression of the LOAD button 66a, the unit 24 energizes all clamps 36, 38, 60 and 64. The operator then installs the set 14 in the fixture 12.

The operator can then open the anticoagulant container 98 permitting a fluid flow to fill the line 76. The operator then depresses the PRIME button 66b. Clamps 38, 60 and 62 automatically close. A manually operated clamp 93 is closed by the operator to prevent fluid flow through line 92. The unit 24 then energizes the bladder 34b, via the line 24f to force saline from the bag 86 through the line 84a, the separator 90 and the lines 85 and 78. After the separator 90 has been filled with saline, clamp 38 is energized to open the line 84 to the flow of saline. When the entire set 14 has been flushed (and bag 86 is empty), and saline has run out of the cannula 70, the operator depresses the RUN button 66c. The system 10 then deenergizes the bladder 34b and closes clamps 36 and 38.

The operator then places the cuff 64 on the arm of the donor D, and the cuff 64 is inflated by the control unit and timer 24. The operator then enters a vein in the arm of the donor D with the sterile cannula 70. This step places the set 14 in a bi-directional fluid flow communication with the donor. This communication is continuously maintained through the following draw and return cycles. The operator then depresses the START button 66d to initiate the first draw and return cycle.

The first draw-return cycle is then commenced and clamps 36, 38, 60 and 62 are automatically opened. The clamp 93 is also opened by the operator. Blood, mixed with anticoagulant, flows under the influence of gravity and the pressure cuff 64 into the container 86. The unit 24 can be set for a predetermined draw cycle; for example, seven minutes. When bag 86 contains the desired amount of whole blood (for example, 500 ml), the housing 34 will prevent additional inflow and no further blood will be drawn from the donor.

Part way through the seven minute draw cycle, clamp 62 is deenergized by the unit 24. The flow of anticoagulant is then decreased during the completion of the draw cycle.

At the end of the seven minute draw cycle, clamps 60 and 38 are deenergized by the unit 24 along with the cuff 64. The bladder 34b is energized by the unit 24. Whole blood is forced through the separator 90. Plasma is collected in the container 94 and simultaneously the concentrated red blood cells are returned to the donor D via the bi-directional fluid flow conduit 72, 78 and the single lumen cannula 70. When the system 10 has completed the return cycle, the clamp 36 is deenergized. The system 10 waits until the operator again presses the START button 66d. Once the START button 66d has been depressed, the unit 24 reinflates the cuff 64 and initiates the next draw cycle.

After the third draw-return cycle the container 94 will contain a desired volume, for example, 500 ml of plasma. Housing 50 limits the separated plasma to the desired volume. In the event the container 94 becomes filled with plasma prior to the end of the third draw cycle, the housing 50 will block further inflow of plasma. The remainder of the whole blood will then be returned to the donor D. The cannula 70 is removed from the arm of the donor D. The operator again depresses the LOAD button 66a. All of the clamps are then energized by the unit 24. The set 14 can then be removed. The plasma container 94 can be removed from the set 14 and sealed as is conventional. The remainder of the set can then be thrown away. The system 10 is then ready for the next donor.

As before stated, the force application system 34b can take the form of a variety of devices to produce the necessary expressing forces.

Preferably the separator 90 will be a hollow membrane fiber filter. It would be desirable from the point of view of optimizing the design of the filter 90 to be able to express the whole blood from the container 86 at a substantially constant, predetermined, pressure.

For purposes of the present disclosure, the phrase "substantially constant predetermined pressure" shall mean a selected, applied pressure that remains essentially constant during the time period during which the whole blood is being forced from the container 86. A pressure variation of 10 or 20% during the first 80 to 90% of the time during which the container 86 is being emptied would still come within the present definition of a substantially constant pressure system.

The system 10 is thus a constant pressure system as opposed to a constant volume system, in which a relatively constant volume of fluid is pumped through the system per unit of time. The system 10, as a constant pressure system, has the further advantage that if a line such as 84a, 85 or 78 becomes crimped or blocked during the return portion of a draw-return cycle, the pressure present therein will not increase as might be the case in a constant volume system.

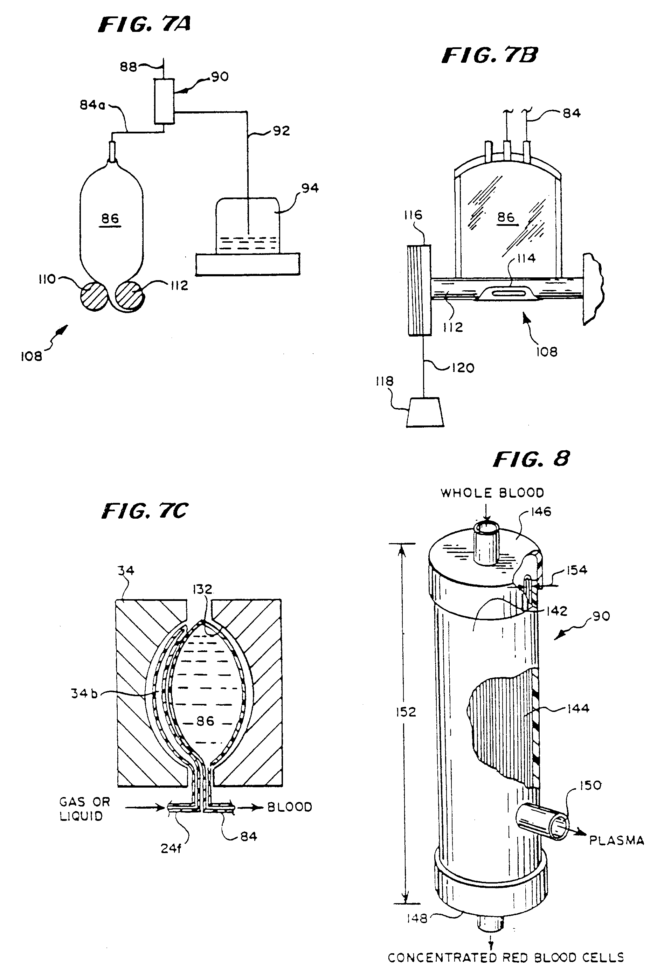

A system 108 which will express the whole blood from the container 86 at a substantially constant predetermined pressure is illustrated in Figures 7A and 7B. This system 108, is formed with a pair of spaced apart rollers 110 and 112.

The rollers 110 and 112 are oriented so as to be parallel with a space there between. For example, the rollers might have a diameter on the order of 1.9 cm (3/4 of an inch) and have an inter-roller gap of 0.3 cm (one eighth of an inch). As illustrated in Figures 7A and 7B, a standard blood collection bag 86 is positioned with a lower tab located in a slot 114 in the roller 112.

In this embodiment, the self-contained energy source for the rollers 110, 112 includes means for releasably storing a quantity of energy to rotate the rollers 110, 112, as well as means for selectively introducing energy into the energy storage means.

While the above-described energy storage and introduction means can be variously constructed, as shown in Fig. 7B, they take the form of a pulley 116 attached to an end of the roller 112. A weight 118 is attached via a flexible cable or line 120 to the pulley 116. By recoiling the line 120 upon the pulley 116 after each use, the pulley 116 can be, in effect, "recharged" for subsequent use.

Experiments have indicated, that notwithstanding the fact that the blood container 86 is flexible and of irregular geometry, as the weight 118 unwinds due to the force of gravity, the force generating apparatus 108 will force the whole blood into the filter 90 at a substantially, constant pressure.

As the blood is forced from the bag 86, the empty portion of the bag 86 is wrapped around the roller 112. As the weight 118 continues to descend from the pulley 116, the bag 86 is continually drawn between the two rollers 110 and 112 and wrapped around the roller 112.

The system 108 will express the whole blood to the filter 90 at a substantially constant pressure on the order of 160 to 180 millimeters of mercury.

To generate pressures in a range of 160-180 mm of mercury, a weight 118 with a mass of 1950 g was used. The pulley 116 had a diameter of 15.9 cm. Larger or smaller pressures can be generated by varying the mass of the weight 118.

As the bag 86 empties, a spike of higher pressure appears after about 80% of the discharge or return period has passed. The effects of the spike can be attenuated by means of an elastic or stretchable, silicone tubing member attached to the weight 118 to slow its rate of descent during the last 20 percent of the discharge time. The presence of this spike does not preclude the roller system 108 from being a generator of substantially constant pressure as that phrase has been defined and used herein.

For example, a WACO 78170-10 elastic silicone tubing member was affixed to the weight 118. The time interval of substantially constant fluid pressure was, as a result, expanded from 80% to 90% of the discharge period. In this instance, a weight 118 with a mass of 4.81 kg was used in combination with a pulley 116 having a diameter of 5.71 cm.

Alternately, instead of using a weight such as the weight 118 as the energy source, a spring which exerts a constant force as it is being extended or as it is being retracted can be used to rotate the pulley 112. Such a spring, with a 3.2 kg (seven pound) force has been used. It has been found experimentally that in connection with the system 108 the use of the constant force 3.2 kg (seven pound) spring results in an output pressure on the order of 140 millimeters of mercury.

In this embodiment, the spring serves as the energy storage device which can be selectively recoiled, thereby "recharged", by the operator for subsequent use.

As an alternate to the roller system 108, and as earlier shown in the system 10 shown in Fig. 5, a commercially available inflatable bladder in a rigid container may be used. In this instance, the housing 34 of Figure 1 corresponds to an external housing of the bladder 34b.

Figure 7C illustrates a system utilizing such a force generating system. An external metal or rigid plastic housing 34 has a cavity 132 defined therein. The inflatable bladder 34b is positioned in the cavity 132. The blood collection bag 86 is placed in the cavity 132. Tubing 24f is provided to inflate the bladder 34b.

The bladder 34b is located adjacent the blood bag 86 and can be inflated by means of pressure from a gas or a liquid. For example, a regulated gas could be used, a liquid CO₂ cartridge could be used, or a gas or liquid under pressure due to a piston could also be used.

As the source of energy inflates the bladder 34b in the housing 34, the blood in the bag 86 is expressed into the tubing 84 at a substantially constant pressure. This pressure can be adjusted to be in a range of 160 to 180 millimeters of mercury as in the case with the dual roller system 108. The pressure should be adjusted in accordance with the resistance of the filter 90 and related flow circuits to provide physiologically acceptable return flow rates in a range of 40 to 80 ml per minute.

A further advantage of the bladder system is that the size of the cavity 132 and bladder 34b limit the volume of blood that can accumulate in the container 86. Hence, after the desired volume of blood has been accumulated in the container 86, the flow of whole blood essentially ceases. Similarly, housing 50 can be used to limit the volume of plasma that accumulates in the container 94.

In addition, unlike the system 108, the bladder system of Figure 7C does not generate a spike of increased pressure and flow rate at the end of the return period. Instead, as the container 86 is emptied at the end of the return cycle, the generated pressure and flow rates decrease to zero.

Figure 8 illustrates an exemplary membrane filter usable with the system 10. The filter 90 includes a hollow cylindrical housing 142 in which is positioned a plurality of hollow fiber membranes 144. The housing 142 includes a blood inlet port 146, a blood outlet port 148 and a plasma output port 150. Fluid flow from the blood outlet port 148 is composed of concentrated red blood cells. This fluid can be regarded as a residual blood component.

The hollow fiber membranes, such as membranes 144, are suitable for contact with human blood and can be formed of polypropylene, polyethylene-covinyl alcohol, nylon, polysulfone or other materials. The fiber members 144 are oriented axially within the housing 142 such that the whole blood flows therethrough from end to end of the filter. The membranes are microporous and contain pores with diameters in a range of 0.1 to 5.0 microns preferably in a range of 0.2 to 0.6 microns.

One consideration in the design of filters such as the filter 90 is minimization of the risk of hemolysis. For given input pressures in a range of 160 to 180 millimeters of mercury, filter parameters of Table I define membrane filters with minimal risk of hemolysis for a given cost.

Each of the six filters defined by Table I includes hollow filtration fibers with each file having a length (L) 152 and an internal diameter (D) 154. The number of fibers (N) defines the number to be axially located in a give housing.

**TABLE I**

| P (mmHg) | N | L (cm) | D (m) |
|---|---|---|---|
| 100 | 3099 | 7.00 | 192 |
| 150 | 2209 | 7.00 | 189 |
| 100 | 2492 | 7.00 | 202 |
| 150 | 1731 | 7.00 | 198 |
| 100 | 1889 | 7.00 | 213 |
| 150 | 1347 | 7.00 | 209 |

Use of a filter with one set of the above parameters will result in extraction of 40 to 70% of the plasma in the whole blood which passes through the filter. Table I also illustrates in each instance the optimized parameters of a hollow membrane filter used in a system with a predetermined pressure drop ( P) between the inlet port 146 and the outlet port 148 of each filter design. As can be seen, the number of fiber members decreases as the pressure drop across the filter is increased. Care must always be taken to insure that the force generating system provides enough pressure at the outlet port 148 to return the filtered blood to the donor D. Thus, for a predetermined pressure drop across the filter, it is possible to optimize the design of the filter. Operating the filter at the predetermined pressure drop optimizes collection of the separated blood component.

The present method and apparatus are particularly advantageous in that, given a substantially constant input pressure, the design and characteristics of the hollow fiber filter 90 can be optimized to provide efficient separation of the plasma component from the whole blood with minimal danger of hemolysis. In addition, the system 10 uses the disposable set 14 that is relatively inexpensive. The system 10 is easy to use and is portable to church basements or recreation halls where blood collection centers are often temporarily established.

The present method and apparatus have been disclosed in exemplary terms of separating and collecting plasma. The present invention, it will be understood, is not limited to plasma collection and separation. The separation and collection of a selected blood component, other than plasma, in accordance with the present apparatus and method are within the spirit and scope of the present invention. For example, leukocytes, lipids, lymphocytes, T-cell subsets, lipoprotein, other lipid moieties, auto-antibodies, immune complexes or the like could be separated and collected in accordance with the present method and apparatus.

## Claims

1. A blood component collection method comprising blood collection and blood separation steps, the blood collection step including accumulating a desired volume of whole blood from a phlebotomy needle (70) through a first conduit (78) in a variable volume container (86), the blood separation step including applying a force in excess of gravity on the container (86) to reduce its volume and thereby express the blood from the container through a second conduit (84a) into a separator (90), separating the blood within the separator into component parts in response, at least in part, to the force applied during the force application step, and collecting at least one of the component parts, characterised in that in the blood collection step all the whole blood that is accumulated in the container is accumulated in response to the internal blood pressure of a donor, without application of suction to the blood in the first conduit (78) and in the blood separation step, none of the blood expressed through the second conduit (84a) and into the separator (90) is accumulated except during the blood collection step in the container (86).

2. A method according to Claim 1 and further including the step of returning at least one of the component parts to the phlebotomy needle (70).

3. A method according to Claim 2, wherein said steps of collecting at least one of the component parts and returning at least one of the component parts occur generally at the same time.

4. A method according to any preceding claim further including the step of introducing anticoagulant into the whole blood as it is being accumulated from the phlebotomy needle (70).

5. A method according to any preceding claim, wherein, during said separation step, a technique involving filtration is used.

6. A method according to any preceding claim and further including the step of returning at least one of the component parts to the phlebotomy needle (70) in response to the force applied during said force application step and without the application of any additional external force.

7. A method according to any preceding claim, wherein the whole blood is expressed from the container (86) at a substantially constant applied pressure.

8. A blood component collection system comprising a variable volume container (86) for accumulating a desired volume of blood from a phlebotomy needle (70), a conduit (78) between the needle (70) and the container (86), a separator (90) for separating blood into component parts, a pathway for conveying blood from the container to the separator, force application means (34b) for applying a force in excess of gravity upon the container to reduce its volume and for expressing blood from the container along the pathway into the separator (90), and collection means (94) for collecting at least one of the component parts, characterised by the absence of a pump which is operable to apply suction to any of the whole blood that is conveyed to the separator (90), the conduit (78) being provided to convey whole blood from the needle to the container in response to the internal blood pressure of a donor, without application of suction to the blood in the conduit.

9. A blood component collection system according to Claim 8, wherein the force application means (34b) is operable to apply a force that conveys the accumulated whole blood into the separator (90) at a pressure that remains substantially constant.

10. A blood component collection system according to Claim 8 or 9 further including means (85,78,72,70) for returning at least one of the component parts from said separation means (90) to the needle (70).

11. A blood component collection system according to Claim 10 wherein said means (85,78,72,70) for returning at least one of the component parts from the separation means (90) to the needle does not include pumping means.

12. A blood component collection system according to any one of Claims 8 to 11 including means (98,62,60,76) for introducing anticoagulant into the whole blood and for metering the anticoagulant into the flow of whole blood at a selected rate as the whole blood is being accumulated in the variable volume container (86).

13. A blood component collection system according to any one of Claims 8 to 12, wherein said separation means (90) includes a filter.

14. A blood component collection system according to any one of Claims 8 to 13 wherein said force application means (34b) includes an inflatable bladder adjacent to said container (86) for applying force thereto.

15. A blood component collection system according to any one of Claims 8 to 14 further including a fixture comprising a housing (34) for releasably retaining said container (86) and force applying means (34b), first means (116) releasably receiving a charge of energy, and second means (112) operatively connected to said first means for releasing said charge of energy and for applying, in response to said released charge of energy, a force upon said container (86) to reduce its volume and express the accumulated blood into the separator (90).

16. A blood component collection system according to Claim 15 and further including means for selectively re-introducing energy into said energy receiving means to recharge said receiving means for subsequent use.

17. A blood component collection system according to Claim 15 wherein said energy receiving means includes a modular charge of energy movable into and out of association with said fixture.

18. A system according to Claim 17 wherein said modular charge of energy includes a replaceable, self-contained cartridge of fluidic energy.

## Patentansprüche

1. Sammelverfahren für Blutbestandteile, das einen Blutsammelschritt und einen Bluttrennschritt aufweist, wobei der Blutsammelschritt das Ansammeln eines gewünschten Vollblutvolumens aus einer Phlebotomienadel (70) durch eine erste Leitung (78) in einem Behälter (86) mit veränderlichem Volumen aufweist und der Bluttrennschritt aufweist: Aufbringen einer Kraft, die größer als die Schwerkraft ist, auf den Behälter (86), um dessen Volumen zu verringern und dadurch das Blut aus dem Behälter durch eine zweite Leitung (84a) in einen Separator (90) auszupressen, Trennen des Bluts in dem Separator in Bestandteile aufgrund mindestens teilweise der während des Kraftaufbringschritts aufgebrachten Kraft und Sammeln mindestens eines der Bestandteile, dadurch gekennzeichnet, daß bei dem Blutsammelschritt das gesamte Vollblut, das in dem Behälter angesammelt wird, aufgrund des inneren Blutdrucks eines Spenders angesammelt wird, ohne daß auf das Blut in der ersten Leitung (78) eine Saugkraft aufgebracht wird, und daß bei dem Bluttrennschritt nichts von dem Blut, das durch die zweite Leitung (84a) und in den Separator (90) ausgepreßt wird, angesammelt wird, mit Ausnahme des Zeitraums während des Schritts des Sammelns von Blut in dem Behälter (86).

2. Verfahren nach Anspruch 1, das ferner den Schritt des Rückführens mindestens eines der Bestandteile zu der Phlebotomienadel (70) aufweist.

3. Verfahren nach Anspruch 2, wobei die Schritte des Sammelns mindestens eines der Bestandteile und des Rückführens mindestens eines der Bestandteile im allgemeinen gleichzeitig stattfinden.

4. Verfahren nach einem der vorhergehenden Ansprüche, das ferner den Schritt des Einführens von Antikoagulans in das Vollblut aufweist, während dieses aus der Phlebotomienadel (70) angesammelt wird.

5. Verfahren nach einem der vorhergehenden Ansprüche, wobei während des Trennschritts eine Technik, die Filtration umfaßt, angewandt wird.

6. Verfahren nach einem der vorhergehenden Ansprüche, das ferner den Schritt des Rückführens mindestens eines der Bestandteile zu der Phlebotomienadel (70) aufgrund der während des Kraftaufbringschritts aufgebrachten Kraft und ohne Aufbringen irgendeiner zusätzlichen äußeren Kraft aufweist.

7. Verfahren nach einem der vorhergehenden Ansprüche, wobei das Vollblut aus dem Behälter (86) mit einem im wesentlichen konstanten aufgebrachten Druck ausgepreßt wird.

8. Sammelsystem für Blutbestandteile, das aufweist: einen Behälter (86) mit veränderlichem Volumen zum Ansammeln eines gewünschten Blutvolumens aus einer Phlebotomienadel (70), eine Leitung (78) zwischen der Nadel (70) und dem Behälter (86), einen Separator (90) zum Trennen von Blut in Bestandteile, eine Bahn zum Transport von Blut aus dem Behälter zu dem Separator, eine Kraftaufbringeinrichtung (34b) zum Aufbringen einer Kraft, die größer als die Schwerkraft ist, auf den Behälter, um dessen Volumen zu verringern, und zum Auspressen von Blut aus dem Behälter entlang der Bahn in den Separator (90) und eine Sammeleinrichtung (94) zum Sammeln mindestens eines der Bestandteile, gekennzeichnet durch das Nichtvorhandensein einer Pumpe, die betätigbar ist, um auf jegliches Vollblut, das zu dem Separator (90) transportiert wird, eine Saugkraft aufzubringen, wobei die Leitung (78) vorgesehen ist, um Vollblut aus der Nadel zu dem Behälter aufgrund des inneren Blutdrucks eines Spenders zu transportieren, ohne daß eine Saugkraft auf das Blut in der Leitung aufgebracht wird.

9. Sammelsystem für Blutbestandteile nach Anspruch 8, wobei die Kraftaufbringeinrichtung (34b) betätigbar ist, um eine Kraft aufzubringen, die das angesammelte Vollblut mit einem Druck, der im wesentlichen konstant bleibt, in den Separator (90) transportiert.

10. Sammelsystem für Blutbestandteile nach Anspruch 8 oder 9, das ferner Mittel (85, 78, 72, 70) zum Rückführen mindestens eines der Bestandteile aus der Trenneinrichtung (90) zu der Nadel (70) aufweist.

11. Sammelsystem für Blutbestandteile nach Anspruch 10, wobei die Mittel (85, 78, 72, 70) zum Rückführen mindestens eines der Bestandteile aus der Trenneinrichtung (90) zu der Nadel keine Pumpeinrichtung aufweisen.

12. Sammelsystem für Blutbestandteile nach einem der Ansprüche 8 bis 11, das Mittel (98, 62, 60, 76) aufweist, um Antikoagulans in das Vollblut einzuführen und um das Antikoagulans in den Vollblutstrom mit einer gewählten Rate zuzumessen, während das Vollblut in dem Behälter (86) mit veränderlichem Volumen angesammelt wird.

13. Sammelsystem für Blutbestandteile nach einem der Ansprüche 8 bis 12, wobei die Trenneinrichtung (90) einen Filter aufweist.

14. Sammelsystem für Blutbestandteile nach einem der Ansprüche 8 bis 13, wobei die Kraftaufbringeinrichtung (34b) eine aufblasbare Blase angrenzend an den Behälter (86) aufweist, um Kraft darauf aufzubringen.

15. Sammelsystem für Blutbestandteile nach einem der Ansprüche 8 bis 14, das ferner eine Vorrichtung hat, die folgendes aufweist: ein Gehäuse (34), um den Behälter (86) und die Kraftaufbringeinrichtung (34b) lösbar festzulegen, eine erste Einrichtung (116), die eine Energieladung freisetzbar aufnimmt, und eine zweite Einrichtung (112), die mit der ersten Einrichtung betriebsmäßig verbunden ist, um die Energieladung freizusetzen und um aufgrund der freigesetzten Energieladung eine Kraft auf den Behälter (86) aufzubringen, um dessen Volumen zu verringern und das angesammelte Blut in den Separator (90) auszupressen.

16. Sammelsystem für Blutbestandteile nach Anspruch 15, das ferner eine Einrichtung zum selektiven Wiedereinleiten von Energie in die Energieaufnahmeeinrichtung aufweist, um die Aufnahmeeinrichtung für den anschließenden Gebrauch erneut zu laden.

17. Sammelsystem für Blutbestandteile nach Anspruch 15, wobei die Energieaufnahmeeinrichtung eine modulare Energieladung aufweist, die in und außer Verbindung mit der Vorrichtung bewegbar ist.

18. System nach Anspruch 17, wobei die modulare Energieladung eine auswechselbare, unabhängige Fluidenergie-Patrone aufweist.

## Revendications

1. Méthode de collecte d'un composant du sang comprenant des étapes de collecte de sang et de séparation de sang, l'étape de collecte de sang incluant l'accumulation d'un volume désiré de sang entier venant d'une aiguille des phlébotomie (70) par l'intermédiaire d'un premier conduit (78) dans un récipient de volume variable (86), l'étape de séparation du sang incluant l'application d'une force supérieure à la pesanteur sur le récipient (86) pour réduire son volume et chasser ainsi le sang du récipient par l'intermédiaire d'un deuxième conduit (84a) vers un séparateur (90), la séparation du sang dans le séparateur en composants en réponse, au moins en partie, à la force appliquée pendant l'étape d'application de force, et la collecte d'au moins un des composants, caractérisée en ce que, dans l'étape de collecte de sang, tout le sang entier qui est accumulé dans le récipient est accumulé en réponse à la pression sanguine interne d'un donneur, sans application d'aspiration au sang dans le premier conduit (78) et, dans l'étape de séparation du sang, aucune partie du sang chassé dans le deuxième conduit (84a) et dans le séparateur (90) n'est accumulée sauf pendant l'étape de collecte du sang dans le récipient (86).

2. Méthode suivant la revendication 1, comprenant en outre l'étape de retour d'au moins un des composants à l'aiguille de phlébotomie (70).

3. Méthode suivant la revendication 2, dans laquelle lesdites étapes de collecte d'au moins un des composants et de retour d'au moins un des composants ont lieu sensiblement en même temps.

4. Méthode suivant une quelconque des revendications précédentes, comprenant en outre l'étape d'introduction d'anti-coagulant dans le sang entier au cours de son accumulation en provenance de l'aiguille de phlébotomie (70).

5. Méthode suivant une quelconque des revendications précédentes, dans laquelle, pendant ladite étape de séparation, on utilise une technique comportant une filtration.

6. Méthode suivant une quelconque des revendications précédentes et comprenant en outre l'étape de retour d'au moins un des composants à l'aiguille de phlébotomie (70) en réponse à la force appliquée pendant ladite étape d'application de force et sans l'application d'une force extérieure additionnelle.

7. Méthode suivant une quelconque des revendications précédentes, dans laquelle le sang entier est expulsé du récipient (86) à une pression appliquée sensiblement constante.

8. Système de collecte d'un composant du sang, comprenant un récipient de volume variable (86) pour accumuler un volume désiré de sang venant d'une aiguille de phlébotomie (70), un conduit (78) entre l'aiguille (70) et le récipient (86), un séparateur (90) pour séparer le sang en composants, un chemin d'amenée du sang du récipient au séparateur, des moyens d'application de force (34b) pour appliquer une force supérieure à la pesanteur sur le récipient afin de réduire son volume et pour chasser le sang du récipient au séparateur (90) par l'intermédiaire dudit chemin, et des moyens de collecte (94) pour collecter au moins un des composants, caractérisé par l'absence d'une pompe servant à appliquer une aspiration à toute partie du sang entier qui est amené au séparateur (90), le conduit (78) étant prévu pour amener le sang entier de l'aiguille au récipient en réponse à la pression sanguine interne d'un donneur, sans application d'aspiration au sang dans le conduit.

9. Système de collecte d'un composant du sang suivant la revendication 8, dans lequel les moyens d'application de force (34b) fonctionnent pour appliquer une force qui déplace le sang entier accumulé vers le séparateur (90)à une pression qui reste sensiblement constante.

10. Système de collecte d'un composant du sang suivant la revendication 8 ou 9, comprenant en outre des moyens (85,78,72,70) pour renvoyer au moins un des composants, desdits moyens de séparation (90) à ladite aiguille (70).

11. Système de collecte d'un composant du sang suivant la revendication 10, dans lequel lesdits moyens (85,78,72,70) pour renvoyer au moins un des composants des moyens de séparation (90) à l'aiguille ne comprennent pas de moyens de pompage.

12. Système de collecte d'un composant du sang suivant une quelconque des revendications 8 à 11, comprenant des moyens (98,62,60,76) pour l'introduction d'anti-coagulant dans le sang entier et pour le dosage de l'anti-coagulant dans l'écoulement de sang entier à un débit choisi, pendant l'accumulation du sang entier dans le récipient de volume variable (86).

13. Système de collecte d'un composant du sang suivant une quelconque des revendications 8 à 12, dans lequel lesdits moyens de séparation (90) comprennent un filtre.

14. Système de collecte d'un composant du sang suivant une quelconque des revendications 8 à 13, dans lequel lesdits moyens d'application de force (34b) comprennent une vessie gonflable adjacente audit récipient (86) pour appliquer une force à ce dernier.

15. Système de collecte d'un composant du sang suivant une quelconque des revendications 8 à 14, comprenant en outre une structure qui comporte un boîtier (34) pour retenir de façon amovible ledit récipient (86) et lesdits moyens d'application de force (34b), des premiers moyens (116) pour recevoir de façon libérable une charge d'énergie, et des deuxièmes moyens (112) fonctionnellement connectés auxdits premiers moyens pour libérer ladite charge d'énergie et pour appliquer, en réponse à ladite charge d'énergie libérée, une force sur ledit récipient (86) afin de réduire son volume et de chasser le sang accumulé vers le séparateur (90).

16. Système de collecte d'un composant du sang suivant la revendication 15, comprenant en outre des moyens pour la réintroduction sélective d'énergie dans les dits moyens de réception d'énergie, afin de recharger les dits moyens de réception pour une utilisation suivante.

17. Système de collecte d'un composant du sang suivant la revendication 15, dans lequel lesdits moyens de réception d'énergie' comprennent une charge modulaire d'énergie qui peut être associée à ladite structure ou enlevée de celle-ci.

18. Système suivant la revendication 17, dans lequel ladite charge modulaire d'énergie comprend une cartouche autonome remplaçable d'énergie fluidique.
